(19) Europäisches Patentamt
European Patent Office
Office européen des brevets

(11) Publication number: **0 098 499**
**A1**

(12) # EUROPEAN PATENT APPLICATION

(21) Application number: **83106255.9**

(22) Date of filing: **27.06.83**

(51) Int. Cl.³: **C 07 D 401/06**

(30) Priority: **06.07.82 IT 2224482**

(43) Date of publication of application: **18.01.84**
**Bulletin 84/3**

(84) Designated Contracting States: **AT BE CH DE FR GB LI LU NL SE**

(71) Applicant: **RAVIZZA S.p.A., 35, Via Europa, I-20053 Muggio' (Milano) (IT)**

(72) Inventor: **Signorini, Roberto, via Pitteri 111, Milan (IT)**
Inventor: **Verga, Alberto, via Tommaso da Cazzaniga 9/6, Milan (IT)**

(74) Representative: **Gervasi, Gemma et al, Studio Brevetti e Marchi NOTARBARTOLO & GERVASI 33, Viale Bianca Maria, I-20122 Milano (IT)**

(54) **Process for preparing ketanserine.**

(57) A process for preparing the compound 3-[2-[4-(p.fluorobenzoyl)-1-piperidinyl]ethyl]-2,4(1H,3H)-quinazolinedione, known in the pharmaceutical field as Ketanserine, by reacting 4-(p.fluorobenzoyl) piperidine with 2,3-dihydro-5H-oxazole (2,3-b)quinazolin-5-one.

EP 0 098 499 A1

PROCESS FOR PREPARING KETANSERINE

This invention relates to a new process for preparing the compound 3-/2-/4-(p.fluorobenzoyl)-1-piperidinyl/ethyl/-2,4(1H,3H)-quinazolinedione, known in the pharmaceutical field by the name of Ketanserine, and used as an anti-hypertensive and anticongestive, particularly in its salified form with pharmacologically suitable acids (The Journal of Pharmacology and Experimental Therapeutics, Vol. 218, No. 1).

The structural formula of the compound is as follows:

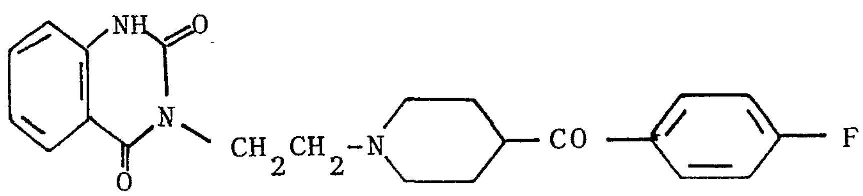

(I)

The known process for preparing the said compound consists of reacting 4-(p.fluorobenzoyl)piperidine with 3-(2-chloroethyl)-2,4 (1H,3H)-quinazolinedione in the presence of $Na_2CO_3$, in a solvent. The yield of this process is very low, being of the order of 20%. Moreover, the presence of extraneous substances in the reaction product, such as sodium carbonate and the solvent, make it necessary to separate and purify the product by industrially costly operations (European Patent Application 80300059.5).

The present invention provides a new process for preparing the product (I), consisting essentially of reacting together equimolecular quantities of 4-(p.fluorobenzoyl)piperidine and 2,3-dihydro-5H-oxazole(2,3-b)quinazolin-5-one.

The reaction scheme is as follows:

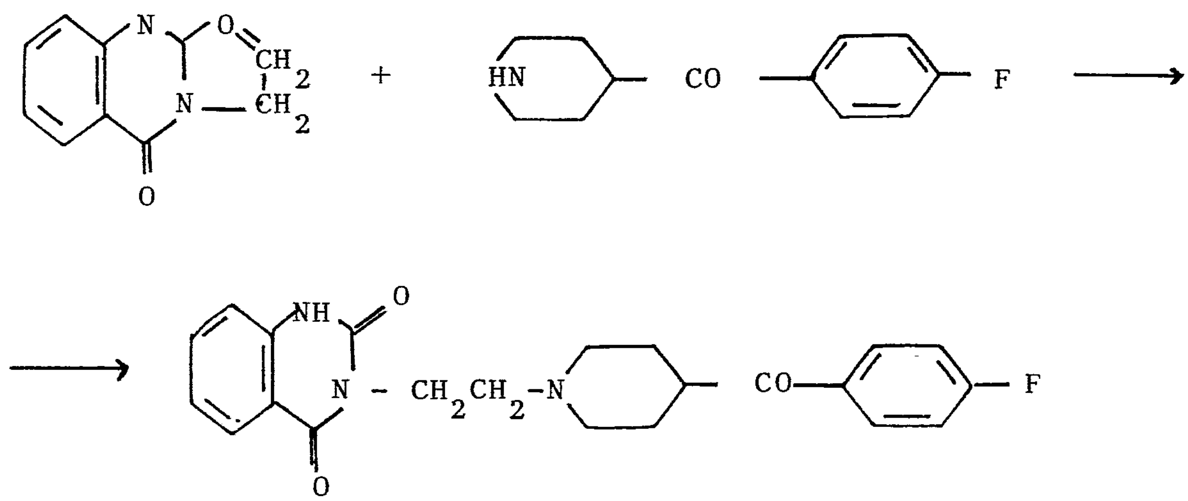

The reaction can be carried out by mixing the two reagents in powder form, and heating the mixture until fusion takes place. The temperature is kept at around 100°C for a certain time to enable the reaction to go to completion. The reaction product is in the form of a solid microcrystalline mass, from which the product can be obtained at a high degree of purity by simply dissolving it in a solvent followed by recrystallisation.

Alternatively, the reaction can be carried out in an inert solvent such as an aromatic hydrocarbon, preferably in the presence of a small quantity of a strong acid acting as catalyst.

Whether the solvent is absent or present, the reaction takes place with a very high yield and conversion. By-products are practically absent, and the crude reaction product contains only a small quantity of unreacted starting substances in addition to the required product (I).

When the Ketanserine has been separated, the reaction solvent or mother liquors of crystallisation can conveniently be recycled to

a further preparation process, as in practice they contain no by-products.

As stated, when carried out in a solvent the reaction can be catalysed by a small quantity of strong acid, which can be either inorganic such as $H_2SO_4$ or HCl, or organic such as a sulphonic acid. In practice an acid can be used having a pK not exceeding 2.

The two reagents used in synthesising the product according to the present invention are known compounds and can be prepared by methods described in the technical literature. Specifically, the preparation of the compound 2,3-dihydro-5H-oxazole(2,3-b) quinazolin-5-one is described by Grout and Partridge in Journal Chemical Soc. (1960) page 3551 onwards.

The preparation of the compound 4-(p.fluorobenzoyl)piperidine is described in Journal of Medicinal Chemistry Vol. 13 No. 1 (1970), page 1 onwards.

The examples given hereinafter illustrate the operating details of the process according to the invention, but without constituting any limitation thereto.

EXAMPLE 1

The following are fed into a flask fitted with a reflux condenser:

| | | |
|---|---|---|
| 4-(p.fluorobenzoyl)piperidine | 20.7 g | 0.1 M |
| 4-(p.fluorobenzoyl)piperidine.HCl | 0.2 g | |
| 2,3-dihydro-5H-oxazole(2,3-b)quinazolin-5-one | 18.8 g | 0.1 M |
| toluene | 200 g | |

The mixture of these substances is heated under reflux, and after 40 minutes a precipitate begins to form and becomes increasingly more dense. After 8 hours, the reaction is almost complete, the

 

mixture is cooled and 31.5 g of 3-/2-/4-(4-fluorobenzoyl)-1-piperidinyl/ethyl/-2,4-(1H,3H)-quinazolinedione (I) are obtained by filtration. Yield 80%.

The mother liquors contain practically only the two starting substances and the product (I), and can be used as such for a subsequent reaction after separating the Ketanserine.

The product (I) is crystallised from dioxane (1:10). M.P. 227°-235° (dec.).

Percentage analysis:

| | | | |
|---|---|---|---|
| calculated | C 66.82 | H 5.60 | N 10.63 |
| found | C 66.88 | H 5.67 | N 10.43 |

EXAMPLE 2

The following are fed into a suitable container:

| | | |
|---|---|---|
| 4-(p.fluorobenzoyl)piperidine | 20.7 g | 0.1 M |
| 2,3-dihydro-5H-oxazole(2,3-b)quinazolin-5-one | 18.8 g | 0.1 M |

The mixture of the two substances in powder form is heated to 100°. Fusion is complete, and after a few minutes the mass solidifies. Heating is continued for 2 hours.

35.5 g of 3-/2-/4(4-fluorobenzoyl)-1-piperidinyl/ethyl/-2,4(1H,3H)-quinazolinedione (I) are obtained. Yield 90%.

The product is crystallised from dioxane (1:10). M.P. 227°-235° (dec.).

The hydrochloride can be prepared from the product obtained in this manner by suspending the base in 70% ethanol and adding the stoichiometric quantity of concentrated hydrochloric acid under hot conditions. The hydrochloride crystallises on cooling.

Yield 90%. M.P. 290°-292° (dec.).

Percentage analysis (hydrochloride monohydrate MW 450):

| | | | |
|---|---|---|---|
| calculated | C 58.66 | H 5.55 | N 9.33 |
| found | C 58.75 | H 5.54 | N 9.39 |

PATENT CLAIMS

1. A process for preparing 3-/2-/4-(p.fluorobenzoyl)-1-piperidinyl/ethyl/-2,4(1H,3H)-quinazolinedione by reacting 4-(p.fluorobenzoyl) piperidine with 2,3-dihydro-5H-oxazole(2,3-b)quinazolin-5-one in equimolecular quantities.

2. A process as claimed in claim 1, wherein the two reagents are heated until the mixture melts, and are kept at this temperature until the reaction is complete.

3. A process as claimed in claim 1, wherein the reaction is carried out in the presence of an inert solvent, under reflux.

4. A process as claimed in claim 3, wherein a small quantity of a strong mineral or organic acid is used as the reaction catalyst.

5. A process as claimed in claim 4, wherein an acid is used having a pK not exceeding 2.

0098499

Application number

European Patent Office

## EUROPEAN SEARCH REPORT

EP 83 10 6255

### DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | JOURNAL OF HETEROCYCLIC CHEMISTRY, vol. 17, November 1980, pages 1553-1557, Hetero Corporation, New York, USA E.P. PAPADOPOULOS: "Reactions of o-aminonitriles with isocyanates. 1. A two-step synthesis of 2,6-dihydroimidazo[1,2-c]quinazolin-5-(3H)one" * Page 1553, line 14; page 1557, column 1, line 27; page 1557 * ----- | 1-5 | C 07 D 401/06 |
| | | | TECHNICAL FIELDS SEARCHED (Int. Cl. 3) |
| | | | C 07 D 401/00<br>C 07 D 498/00 |

The present search report has been drawn up for all claims

| Place of search | Date of completion of the search | Examiner |
|---|---|---|
| THE HAGUE | 03-10-1983 | FRANCOIS J.C.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82